(19) Europäisches Patentamt
European Patent Office
Office européen des brevets

(11) EP 1 397 366 B1

(12) EUROPEAN PATENT SPECIFICATION

(45) Date of publication and mention of the grant of the patent:
**07.02.2007 Bulletin 2007/06**

(21) Application number: **02731063.0**

(22) Date of filing: **29.05.2002**

(51) Int Cl.:
***C07D 491/22*** *(2006.01)* ***A61K 31/439*** *(2006.01)*
***A61P 25/00*** *(2006.01)* ***A61P 1/04*** *(2006.01)*

(86) International application number:
**PCT/SE2002/001031**

(87) International publication number:
**WO 2002/096912 (05.12.2002 Gazette 2002/49)**

(54) **NOVEL LIGAND FOR NICOTINIC ACETYLCHOLINE RECEPTORS USEFUL IN THERAPY**

NEUER, FÜR DIE THERAPIE GEEIGNETER LIGAND FÜR NIKOTINISCHE ACETYLCHOLINREZEPTOREN

NOUVEAU LIGAND POUR RECEPTEURS NICOTINIQUES DE L'ACETYLCHOLINE UTILISES EN THERAPIE

(84) Designated Contracting States:
**AT BE CH CY DE DK ES FI FR GB GR IE IT LI LU MC NL PT SE TR**
Designated Extension States:
**SI**

(30) Priority: **01.06.2001 US 295206 P**

(43) Date of publication of application:
**17.03.2004 Bulletin 2004/12**

(73) Proprietor: **AstraZeneca AB**
**151 85 Södertälje (SE)**

(72) Inventor: **PHILLIPS, Eifion**
**Wilmington, DE 19850-5437 (US)**

(56) References cited:
**WO-A1-00/42044** **WO-A1-98/54189**
**WO-A1-99/03859**

- **NORDVALL GUNNAR ET AL.: '3-(2-Benzofuranyl) quinuclidin-2-ene derivatives: Novel muscarinic antagonists' J. MED. CHEM. vol. 39, 1996, pages 3269 - 3277, XP002123211**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 397 366 B1

## Description

### Technical Field

This invention relates to novel spiroazabicyclic heterocyclic amines or pharmaceutically acceptable salts thereof, processes for preparing them, pharmaceutical compositions containing them and their use in therapy. A further object is to provide active compounds which are potent ligands for nicotinic acetylcholine receptors (nAChR's).

### Background Of The Invention

The use of compounds which bind nicotinic acetylcholine receptors in the treatment of a range of disorders involving reduced cholinergic function such as Alzheimer's disease, cognitive or attention disorders, anxiety, depression, smoking cessation, neuroprotection, schizophrenia, analgesia, Tourette's syndrome, and Parkinson's disease has been discussed in McDonald et al. (1995) "Nicotinic Acetylcholine Receptors: Molecular Biology, Chemistry and Pharmacology", Chapter 5 in Annual Reports in Medicinal Chemistry, vol. 30, pp. 41-50, Academic Press Inc., San Diego, CA; and in Williams et al. (1994) "Neuronal Nicotinic Acetylcholine Receptors," Drug News & Perspectives, vol. 7, pp. 205-223.

US Patent 5,468,875 discloses N-alkylcarbamic acid 1-azabicyclo[2.2.1]hept-3-yl esters which are centrally active muscarinic agents useful in the treatment of Alzheimer's disease and other disorders.

N-(2-alkoxyphenyl)carbamic acid 1-azabicyclo [2.2.2]octan-3-yl esters are disclosed in Pharmazie, vol. 48, 465-466 (1993) along with their local anesthetic activity. N-phenylcarbamic acid 1-azabicyclo[2.2.2]octan-3-yl esters substituted at the *ortho* position on the phenyl ring are described as local anaesthetics in *Acta Pharm. Suecica,* 7, 239-246 (1970). Furopyridines useful in controlling synaptic transmission are disclosed in WO 97/05139.

### Summary of the Invention

The invention generally relates to a compound having the formula:

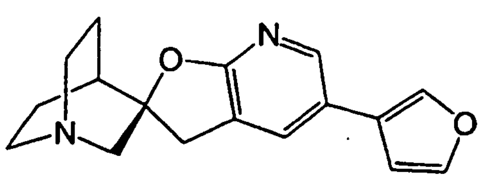

,

and any pharmaceutically-acceptable salts thereof, and their uses in therapy and compositions containing them.

### Disclosure Of The Invention

One aspect of the invention is A compound having the formula:

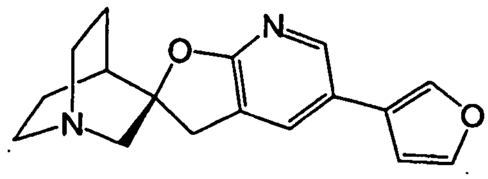

,

and any pharmaceutically-acceptable salts thereof.

Another aspect of the invention relates to a pharmaceutical composition comprising a compound as described above, and a pharmaceutically-acceptable diluent or carrier.

Another aspect of the invention relates to the above pharmaceutical composition for use in the treatment of prophylaxis of human diseases or conditions in which activation of the $\alpha$7 nicotinic receptor beneficial.

Another aspect of the invention relates to the above pharmaceutical composition for use in the treatment or prophylaxis of psychotic disorders or intellectual impairment disorders.

Another aspect of the invention relates to the above pharmaceutical composition for use in the treatment or prophylaxis of Alzheimer's disease, learning deficit, cognition deficit, attention deficit, memory loss, Attention Deficit Hyperactivity Disorder, anxiety, schizophrenia, or mania or manic depression Parkinson's disease, Huntington's disease, Tourette's syndrome, neurodegenerative disorders in which there is loss of cholinergic synapse, jetlag, cessation of smoking, nicotine addiction including that resulting from exposure to products containing nicotine, pain, and for ulcerative colitis.

Another aspect of the invention relates to a use of a compound as described above in the manufacture of a medicament for the treatment or prophylaxis of human diseases or conditions in which activation of the $\alpha$7 nicotinic receptor is beneficial.

Another aspect of the invention relates to a use of a compound as described above in the manufacture of a medicament for the treatment or prophylaxis of psychotic disorders or intellectual impairment disorders.

Another aspect of the invention relates to the above use, wherein the condition or disorder is Alzheimer's disease, learning deficit, cognition deficit, attention deficit, memory loss, Attention Deficit Hyperactivity Disorder.

Another aspect of the invention relates to the above use, wherein the disorder is anxiety, schizophrenia, or mania or manic depression.

Another aspect of the invention relates to the above use, wherein the disorder is Parkinson's disease, Huntington's disease, Tourette's syndrome, or neurodegenerative disorders in which there is loss of cholinergic

synapses.

Another aspect of the invention relates to the use of a compound as described above in the manufacture of a medicament for the treatment or prophylaxis of jetlag, cessation of smoking, nicotine addiction including that resulting from exposure to products containing nicotine, pain, and for ulcerative colitis.

## Pharmaceutical Compositions

A further aspect of the invention relates to a pharmaceutical composition for treating or preventing a condition or disorder as exemplified below arising from dysfunction of nicotinic acetylcholine receptor neurotransmission in a mammal, preferably a human, comprising an amount of a compound of formula I, an enantiomer thereof or a pharmaceutically acceptable salt thereof, effective in treating or preventing such disorder or condition and an inert pharmaceutically acceptable carrier.

For the above-mentioned uses the dosage administered will, of course, vary with the compound employed, the mode of administration and the treatment desired. However, in general, satisfactory results are obtained when the compounds of the invention are administered at a daily dosage of from about 0.1 mg to about 20 mg per kg of animal body weight, preferably given in divided doses 1 to 4 times a day or in sustained release form. For man, the total daily dose is in the range of from 5 mg to 1,400 mg, more preferably from 10 mg to 100 mg, and unit dosage forms suitable for oral administration comprise from 2 mg to 1,400 mg of the compound admixed with a solid or liquid pharmaceutical carrier or diluent.

The compounds of formula I, or an enantiomer thereof, and pharmaceutically acceptable salts thereof, may be used on their own or in the form of appropriate medicinal preparations for enteral or parenteral administration. According to a further aspect of the invention, there is provided a pharmaceutical composition including preferably less than 80% and more preferably less than 50% by weight of a compound of the invention in admixture with an inert pharmaceutically acceptable diluent or carrier. Examples of diluents and carriers are:

- for tablets and dragees: lactose, starch, talc, stearic acid; for capsules: tartaric acid or lactose;
- for injectable solutions: water, alcohols, glycerin, vegetable oils; for suppositories: natural or hardened oils or waxes.

There is also provided a process for the preparation of such a pharmaceutical composition which comprises mixing the ingredients.

## Utility

A further aspect of the invention is the use of a compound according to the invention, an enantiomer thereof or a pharmaceutically acceptable salt thereof, in the manufacture of a medicament for the treatment or prophylaxis of one of the below mentioned diseases or conditions; and a method of treatment or prophylaxis of one of the above mentioned diseases or conditions, which comprises administering a therapeutically effective amount of a compound according to the invention, or an enantiomer thereof or a pharmaceutically acceptable salt thereof, to a patient.

Compounds according to the invention are agonists of nicotinic acetylcholine receptors. While not being limited by theory, it is believed that agonists of the α7 nAChR (nicotinic acetylcholine receptor) subtype should be useful in the treatment or prophylaxis of psychotic disorders and intellectual impairment disorders, and have advantages over compounds which are or are also agonists of the α4 nAChR subtype. Therefore, compounds which are selective for the α7 nAChR subtype are preferred. The compounds of the invention are indicated as pharmaceuticals, in particular in the treatment or prophylaxis of psychotic disorders and intellectual impairment disorders. Examples of psychotic disorders include schizophrenia, mania and manic depression, and anxiety. Examples of intellectual impairment disorders include Alzheimer's disease, learning deficit, cognition deficit, attention deficit, memory loss, and Attention Deficit Hyperactivity Disorder. The compounds of the invention may also be useful as analgesics in the treatment of pain (including chronic pain) and in the treatment or prophylaxis of Parkinson's disease, Huntington's disease, Tourette's syndrome, and neurodegenerative disorders in which there is loss of cholinergic synapses. The compounds may further be indicated for the treatment or prophylaxis of jetlag, for use in inducing the cessation of smoking, and for the treatment or prophylaxis of nicotine addiction (including that resulting from exposure to products containing nicotine).

It is also believed that compounds according to the invention are useful in the treatment and prophylaxis of ulcerative colitis.

## Pharmacology

The pharmacological activity of the compounds of the invention may be measured in the tests set out below:

## Test A - Assay for affinity at α7 nAChR subtype

$_{125}$I-α-Bungarotoxin (BTX) binding to rat hippocampal membranes. Rat hippocampi were homogenized in 20 volumes of cold homogenization buffer (HB: concentrations of constituents (mM): tris(hydroxymethyl) aminomethane 50; $MgCl_2$ 1; NaCl 120; KCl 5: pH 7.4). The homogenate was centrifuged for 5 minutes at 1000 g, the supematant was saved and the pellet re-extracted. The pooled supernatants were centrifuged for 20 minutes at 12000 g, washed, and resuspended in HB. Mem-

branes (30-80 $\mu$g) were incubated with 5 nM [$^{125}$I]$\alpha$-BTX, I mg/mL BSA (bovine serum albumin), test drug, and either 2 mM $CaCl_2$ or 0.5 mM EGTA [ethylene glycol-bis ($\beta$-aminoethylether)] for 2 hours at 21°C, and then filtered and washed 4 times over Whatman glass fibre filters (thickness C) using a Brandel cell harvester. Pretreating the filters for 3 hours with 1% (BSA/0.01% PEI (polyethyleneimine) in water was critical for low filter blanks (0.07% of total counts per minute). Nonspecific binding was described by 100 $\mu$M (-)-nicotine, and specific binding was typically 75%.

**Test B - Assay for affinity to the $\alpha$4 nAChR subtype**

[$^3$H]-(-)-nicotine binding. Using a procedure modified from Martino-Barrows and Kellar (Mol Pharm (1987) 31:169-174), rat brain (cortex and hippocampus) was homogenized as in the [$_{125}$I]$\alpha$-BTX binding assay, centrifuged for 20 minutes at 12,000 x g, washed twice, and then resuspended in HB containing 100 $\mu$M diisopropyl fluorophosphate. After 20 minutes at 4°C, membranes (approximately 0.5 mg) were incubated with 3 nM [3H]-(-)-nicotine, test drug, 1 $\mu$M atropine, and either 2 mM $CaCl_2$ or 0.5 mM EGTA for 1 hour at 4°C, and then filtered over Whatman glass fibre filters (thickness C) (pretreated for 1 hour with 0.5% PEI) using a Brandel cell harvester. Nonspecific binding was described by 100 $\mu$M carbachol, and specific binding was typically 84%.

**Binding data analysis for Tests A and B**

$IC_{50}$ values and pseudo Hill coefficients ($n_H$) were calculated using the non-linear curve fitting program ALLFIT (DeLean A, Munson P J and Rodbard D (1977) Am. J. Physiol., 235:E97-E102). Saturation curves were fitted to a one site model, using the non-linear regression program ENZFITTER (Leatherbarrow, R.J. (1987)); yielding $K_D$ values of 1.67 and 1.70 nM for the $^{125}$I-$\alpha$-BTX and [$^3$H]-(-)-nicotine ligands respectively. $K_i$ values were estimated using the general Cheng-Prusoff equation:

$$K_i\text{-}[IC_{50}]/((2+([\text{ligand}]/[K_D])_n)_{1/n}\text{-}1)$$

where a value of n=1 was used whenever $n_H$<1.5 and a value of n=2 was used when $n_H \geq 1.5$. Samples were assayed in triplicate and were typically $\pm$5%. $K_i$ values were determined using 6 or more drug concentrations. The compounds of the invention are compounds with binding affinities ($K_i$) of less than 1000 nM in either Test A or Test B, indicating that they are expected to have useful therapeutic activity.

The compounds of the invention have the advantage that they may be less toxic, be more efficacious, be longer acting, have a broader range of activity, be more potent, produce fewer side effects, are more easily absorbed or have other useful pharmacological properties.

**Examples**

Commercial reagents were used without further purification. Mass spectra were recorded using either a Hewlett Packard 5988A or a MicroMass Quattro-1 Mass Spectrometer and are reported as m/z for the parent molecular ion with its relative intensity. Room temperature refers to 20-25°C.

For additional examples and precursors, see published application WO 99/03859.

**Example 1A**

5'-Bromospiro[1-azabicyclo[2.2.2]octane-3,2'(3'H)-furo[2,3-b]pyridine] A solution of spiro[1-azabicyclo[2.2.2]octane-3,2'(3'H)-furo[2,3-b]pyridine] (100 mg, 0.462 mmol) and sodium acetate (410 mg, 5 mmol) in 50 % aqueous acetic acid (4mL) was heated to 60°C. Bromine (0.100 mL, 1.94 mmol) was added via a syringe over 10 minutes, and the solution was then heated under reflux for 1 hour. The mixture was allowed to cool to ambient temperature, basified to pH >10 with sodium carbonate, and extracted with chloroform (3 x 15 mL). The combined extracts were dried ($MgSO_4$), filtered, and evaporated under reduced pressure to give the title compound (110 mg, 0.37 mmol, 81 %) as an off-white solid: electrospray MS 295 ([MH]$^+$, with $^{79}$Br, 100), 297 ([MH]$^+$, with $^{81}$Br, 98).

**Example 1B**

(R)-(-)-5'-Bromospiro[1-azabicyclo[2.2.2]octane-3,2'(3'H)-furo[2,3-b]pyridine] The enantiomer (R)-(-)-spiro [1- azabicyclo [2.2.2] octane- 3,2' (3'H)-furo [2,3-b]pyridine] (1.95 g, 9 mmol) treated in the same way as described in example 2A provided the title compound (1.77 g, 6 mmol, 67%) ([$\alpha$]$^{23}$ = -45.5 ° (c = 1, MeOH)).

**Example 2**

(2'R)-5'-(3-furanyl)spiro[1-azabicyclo[2.2.2]octane-3,2'(3'H)-furo[2,3-b]pyridine]

(2'R)- 5'- bromo- spiro [1- azabicyclo [2.2.2] octane-3,2'(3'H)-furo[2,3-b]pyridine] (0.70 g, 2.37 mmol), 3-furylboronic acid (0.39 g, 3.5 mmol), tetrakis(triphenylphosphine)palladium (0) (131 mg, 0.11 mmol), and sodium carbonate (0.75 g, 7.1 mmol) were placed in a tube under nitrogen. Water (3 ml), ethanol (3 ml) and tetrahydrofuran (12 ml) were added. The mixture was then heated at 70 °C and stirred under nitrogen for 24 h. The mixture was then evaporated under vacuum and the residue from evaporation was partitioned between dilute aqueous sodium hydroxide and chloroform, the layers were separated, and the aqueous layer was further extracted

with chloroform. The chloroform extract was dried (magnesium sulfate), filtered, and evaporated. The residue was purified by reverse phase HPLC on a Waters Novapak-HR $C_{18}$ Column using a gradient of 0-70% acetonitrile / water (each solvent containing 0.1% trifluoroacetic acid as a buffer) as the eluant. The product-containing fractions were evaporated, then the residue was dissolved in methanol. Excess concentrated hydrochloric acid was added, and the solution was evaporated to give the dihydrochloride salt of the title compound (489 mg) as a colourless solid; m.p. 223-225 ˚C (decomp.); m/z 283 (100%, MH+).

## Claims

1. A compound having the formula:

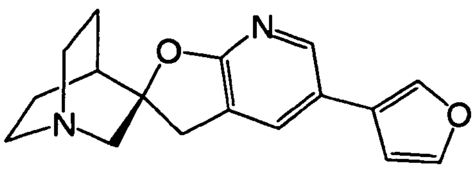

,

and any pharmaceutically-acceptable salts thereof.

2. A pharmaceutical composition comprising a compound as described in Claim 1, and a pharmaceutically-acceptable diluent or carrier.

3. The pharmaceutical composition according to Claim 2 for use in the treatment of prophylaxis of human diseases or conditions in which activation of the α7 nicotinic receptor beneficial.

4. The pharmaceutical composition according to Claim 2 for use in the treatment or prophylaxis of psychotic disorders or intellectual impairment disorders.

5. The pharmaceutical composition according to Claim 2 for use in the treatment or prophylaxis of Alzheimer's disease, learning deficit, cognition deficit, attention deficit, memory loss, Attention Deficit Hyperactivity Disorder, anxiety, schizophrenia, or mania or manic depression Parkinson's disease, Huntington's disease, Tourette's syndrome, neurodegenerative disorders in which there is loss of cholinergic synapse, jetlag, cessation of smoking, nicotine addiction including that resulting from exposure to products containing nicotine, pain, and for ulcerative colitis.

6. Use of a compound as described in Claim 1 in the manufacture of a medicament for the treatment or prophylaxis of human diseases or conditions in which activation of the α7 nicotinic receptor is beneficial.

7. Use of a compound as described in Claim 1 in the manufacture of a medicament for the treatment or prophylaxis of psychotic disorders or intellectual impairment disorders.

8. The use according to Claim 7, wherein the condition or disorder is Alzheimer's disease, learning deficit, cognition deficit, attention deficit, memory loss, Attention Deficit Hyperactivity Disorder.

9. The use according to Claim 7 wherein the disorder is anxiety, schizophrenia, or mania or manic depression.

10. The use as claimed in claim 7 wherein the disorder is Parkinson's disease, Huntington's disease, Tourette's syndrome, or neurodegenerative disorders in which there is loss of cholinergic synapses.

11. Use of a compound as described in Claim 1 in the manufacture of a medicament for the treatment or prophylaxis of jetlag, cessation of smoking, nicotine addiction including that resulting from exposure to products containing nicotine, pain, and for ulcerative colitis.

12. A compound as described in Claim 1 for use as a medicament.

## Patentansprüche

1. Verbindung der Formel:

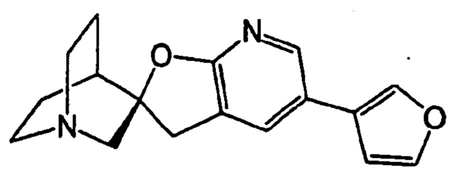

,

und pharmazeutisch annehmbare Salze davon.

2. Pharmazeutische Zusammensetzung, enthaltend eine Verbindung gemäß Anspruch 1 und ein pharmazeutisch annehmbares Verdünnungsmittel oder einen pharmazeutisch annehmbaren Träger.

3. Pharmazeutische Zusammensetzung nach Anspruch 2 zur Verwendung bei der Behandlung oder Prophylaxe von Erkrankungen oder Zuständen des Menschen, bei denen die Aktivierung des nicotinischen Rezeptors α7 vorteilhaft ist.

4. Pharmazeutische Zusammensetzung nach Anspruch 2 zur Verwendung bei der Behandlung oder Prophylaxe von psychotischen Störungen oder Intelligenzstörungen.

5. Pharmazeutische Zusammensetzung nach Anspruch 2 zur Verwendung bei der Behandlung oder Prophylaxe von Alzheimer-Krankheit, Lernschwäche, Denkschwäche, Konzentrationsstörungen, Gedächtnisschwund, hyperkinetischem Syndrom, Angst, Schizophrenie, Manie oder manischer Depression, Parkinson-Krankheit, Chorea Huntington, Tourette-Syndrom, neurodegenerativen Erkrankungen mit Verlust cholinerger Synapsen, Jetlag, Raucherentwöhnung, Nicotinabhängigkeit einschließlich der sich aus der Exposition gegenüber nicotinhaltigen Produkten ergebenden Abhängigkeit, Schmerzen und Colitis ulcerosa.

6. Verwendung einer Verbindung gemäß Anspruch 1 bei der Herstellung eines Arzneimittels zur Behandlung oder Prophylaxe von Erkrankungen oder Zuständen des Menschen, bei denen die Aktivierung des nicotinischen Rezeptors α7 vorteilhaft ist.

7. Verwendung einer Verbindung gemäß Anspruch 1 bei der Herstellung eines Arzneimittels zur Behandlung oder Prophylaxe von psychotischen Störungen oder Intelligenzstörungen.

8. Verwendung nach Anspruch 7, bei der es sich bei dem Zustand oder der Erkrankung um Alzheimer-Krankheit, Lernschwäche, Denkschwäche, Konzentrationsstörungen, Gedächtnisschwund oder hyperkinetisches Syndrom handelt.

9. Verwendung nach Anspruch 7, bei der es sich bei der Erkrankung um Angst, Schizophrenie, Manie oder manische Depression handelt.

10. Verwendung nach Anspruch 7, bei der es sich bei der Erkrankung um Parkinson-Krankheit, Chorea Huntington, Tourette-Syndrom oder neurodegenerative Erkrankungen mit Verlust cholinerger Synapsen handelt.

11. Verwendung einer Verbindung gemäß Anspruch 1 bei der Herstellung eines Arzneimittels zur Behandlung oder Prophylaxe von Jetlag, Raucherentwöhnung, Nicotinabhängigkeit einschließlich der sich aus der Exposition gegenüber nicotinhaltigen Produkten ergebenden Abhängigkeit, Schmerzen und Colitis ulcerosa.

12. Verbindung gemäß Anspruch 1 zur Verwendung als Arzneimittel.

**Revendications**

1. Composé présentant la formule :

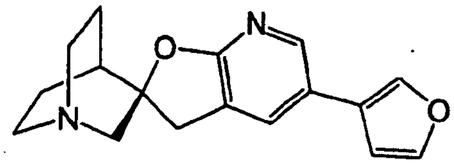

,

et de quelconques sels pharmaceutiquement acceptables de celui-ci.

2. Composition pharmaceutique comprenant un composé tel que décrit à la revendication 1 et un diluant ou un support pharmaceutiquement acceptable.

3. Composition pharmaceutique selon la revendication 2, destinée à une utilisation dans le traitement ou la prophylaxie de maladies humaines ou d'états dans lesquels l'activation du récepteur α7-nicotinique est bénéfique.

4. Composition pharmaceutique selon la revendication 2, destinée à une utilisation dans le traitement ou la prophylaxie de troubles psychotiques ou de troubles de diminution intellectuelle.

5. Composition pharmaceutique selon la revendication 2, destinée à une utilisation dans le traitement ou la prophylaxie de la maladie d'Alzheimer, d'un déficit d'apprentissage, d'un déficit cognitif, d'un déficit de l'attention, de la perte de mémoire, d'un déficit de concentration lié un trouble d'hyperactivité, de l'anxiété, de la schizophrénie, ou de la folie ou de la maniaco-dépression, de la maladie de Parkinson, de la maladie de Huntington, du syndrome de Tourette, de troubles de neurodégénérescence dans lesquels il existe une perte du synapse cholinergique, du décalage horaire, de l'arrêt de la cigarette, de la dépendance à la nicotine, comprenant celle provenant d'exposition à des produits contenant de la nicotine, de la douleur et de la colite ulcéreuse.

6. Utilisation d'un composé tel que décrit à la revendication 1 dans la préparation d'un médicament destiné au traitement ou à la prophylaxie de maladies humaines ou d'états dans lesquels l'activation du récepteur α7-nicotinique est bénéfique.

7. Utilisation d'un composé tel que décrit à la revendication 1 dans la préparation d'un médicament destiné au traitement ou à la prophylaxie de troubles psychotiques ou de troubles de diminution intellectuelle.

**8.** Utilisation selon la revendication 7, dans laquelle l'état ou le trouble est la maladie d'Alzheimer, un déficit d'apprentissage, un déficit cognitif, un déficit de l'attention, une perte de mémoire, un déficit de concentration lié à un trouble d'hyperactivité.

**9.** Utilisation selon la revendication 7, dans laquelle le trouble est l'anxiété, la schizophrénie, ou la folie ou la maniaco-dépression.

**10.** Utilisation selon la revendication 7, dans laquelle le trouble est la maladie de Parkinson, la maladie de Huntington, le syndrome de Tourette, ou des troubles de neurodégénérescence dans lesquels il existe une perte des synapses cholinergiques.

**11.** Utilisation d'un composé tel que décrit à la revendication 1 dans la préparation d'un médicament destiné au traitement ou à la prophylaxie du décalage horaire, de l'arrêt de la cigarette, de la dépendance à la nicotine, y compris celle qui résulte d'une exposition à des produits contenant de la nicotine, de la douleur et de la colite ulcéreuse.

**12.** Composé tel que décrit à la revendication 1, destiné à une utilisation en tant que médicament.